# FASCICULE DE BREVET EUROPEEN

(11) **EP 0 678 284 B1**
(45) Date de publication et mention de la délivrance du brevet: **04.09.2002**
(21) Numéro de dépôt: 95400850.4
(22) Date de dépôt: 14.04.1995
(51) Int. Cl.: A61F 2/01

(54) **Filtre sanguin à usage temporaire ou définitif et son dispositif d'implantation,**
Temporärer oder permanenter Blutfilter und Implantierungsvorrichtung dafür
Blood filter for temporary or permanent use, and associated implanting device

(30) Priorité: 21.04.1994 FR 9404804
(43) Date de publication de la demande: 25.10.1995
(60) Demande divisionnaire: 02000785.2
(73) Titulaire: B. Braun Medical SAS, 92100 Boulogne Billancourt (FR)
(72) Inventeur: Chevillon, Gérard, F-92120 Montrouge (FR); Nadal, Guy, F-86000 Poitiers (FR); Iachetti, Massimo, I-00199 Roma (IT)
(74) Mandataire: Lerner, François

(56) Documents cités:
- EP-A- 0 348 295
- EP-A- 0 582 493
- FR-A- 2 573 646
- FR-A- 2 587 901
- FR-A- 2 649 884
- US-A- 4 425 908

## Description

L'invention a pour objet un ensemble comprenant un filtre médical destiné à être positionné dans un vaisseau sanguin pour piéger des caillots pouvant y circuler et son dispositif d'implantation.

Pour implanter ces filtres dans le corps d'un patient, le praticien utilise habituellement la voie endoveineuse, en pratiquant soit une dénudation vasculaire soit en suivant une technique d'introduction dite percutanée.

La technique percutanée est la moins agressive car un dispositif du type introducteur de cathéter peut être utilisé pour l'introduction du filtre par voie jugulaire ou fémorale. Après avoir pratiqué une incision de la peau, le filtre est ainsi introduit dans la veine retenue, puis est guidé jusqu'à la veine cave inférieure où il est "expulsé" hors du dispositif. Il s'expanse ainsi dans la veine, en prévenant la migration des caillots qui se dirigent vers le coeur et l'artère pulmonaire et évitant l'embolie.

Suivant les risques, deux grandes catégories de filtres sont aujourd'hui disponibles.

Face tout d'abord à des risques d'embolie graves, on propose des filtres dits "définitifs", c'est-à-dire prévus pour être implantés de façon permanente.

De tels filtres sont par exemple décrits dans les brevets US-A-3 540 431, US-A-4 688 553 ou encore dans FR-A-2 689 388.

Etant implanté de façon définitive, ces filtres doivent être fixés à la veine et comportent souvent pour cela des crochets qui s'ancrent dans la paroi du vaisseau au moment où le filtre est lâché et s'expanse radialement (souvent de lui-même, nombre de filtres étant "auto-expansibles").

Dans certain cas, il arrive toutefois que les risques d'embolie pulmonaire ne nécessitent pas un traitement de filtration in situ permanent. Face à cela, on a déjà proposé des filtres dits "temporaires" (par exemple décrits dans FR-A-2 694 687 ou FR-A-2 657 261), c'est-à-dire présentant une structure telle qu'ils puissent être retirés après avoir été implantés.

Ces filtres temporaires ou définitifs présentent l'inconvénient d'être sélectifs, c'est-à-dire que si le praticien juge d'abord nécessaire l'utilisation d'un filtre temporaire puis juge dans un second temps qu'il faut un filtre définitif, il doit impérativement retirer le premier puis implanter le second. Dans le cas inverse, s'il a implanté d'emblée un filtre définitif, il est trop tard pour songer à utiliser un filtre temporaire.

Face à la difficulté parfois grande pour le praticien d'évaluer d'emblée l'importance du danger d'embolie, on a proposé dans EP-A-0 348 295 un ensemble de filtration à usage temporaire et/ou définitif qui comprend, d'une part, un filtre à pattes flexibles définissant deux corolles tête-bêche (l'une de filtration et l'autre de maintien du filtre en implantation définitive dans le vaisseau) et, d'autre part, un double-cathéter de maintien du filtre en implantation temporaire. En usage temporaire, le filtre est maintenu lié au double-cathéter simplement par frottement des pattes de sa corolle de maintien contre la paroi du cathéter externe.

En utilisation définitive, le filtre se trouve complètement expansé dans le vaisseau, avec sa corolle de maintien en appui contre la paroi du vaisseau pour y fixer le filtre, le double-cathéter étant normalement retiré du corps du patient.

En pratique, cet ensemble s'est s'avéré ne pas toujours présenter une fiabilité totale et être d'une manipulation complexe et délicate pour l'implantation du filtre. En effet, on notera en particulier que :
- trois tubes coaxiaux (gaine et double-cathéter) sont nécessaires pour l'implantation correcte du filtre, ce qui augmente l'encombrement (section) du système et complique son introduction dans des voies sinueuses ou étroites,
- tout mouvement accidentel du cathéter interne par rapport au cathéter externe provoquerait un déplacement non souhaité du filtre à l'intérieur de cathéter externe (pouvant fragiliser le maintien du filtre) voire une expulsion du filtre hors de cette gaine,
- le cathéter interne agit sur le filtre, à l'extrémité proximale de celui-ci et à l'écart de la tête, ce qui ne rend pas toujours son déplacement aisé,
- de plus, le cathéter interne déplace le filtre uniquement dans le sens de son expulsion hors du cathéter externe, ce qui ne permet pas toujours un contrôle précis du déplacement du filtre, notamment pour un bon positionnement dans le vaisseau.

Pour contrôler quelque peu l'éjection d'un filtre définitif hors de sa gaine d'implantation, il a déjà été proposé dans FR-A-26 45 028, de pourvoir un système classique d'implantation de filtres définitifs de moyens de liaison amovible entre la tige-poussoir et l'extrémité proximale du filtre, cette tige présentant alors une extrémité distale évidée pour recevoir la tête du filtre et la serrer tant qu'elle est étroitement disposée à l'intérieur de la gaine.

Il s'avère, toutefois qu'à la connaissance de la demanderesse, qu'il n'a jamais été proposé à ce jour un ensemble de filtration temporaire et/ou définitive de structure simple, fiable et comprenant des moyens de liaison amovible prévus sur la tige-poussoir et sur le filtre.

Face à cela et en vue de concilier les avantages des filtres temporaires et des filtres définitifs, l'invention propose une solution qui permet de faire évoluer le filtre une fois implanté d'une utilisation temporaire vers une utilisation définitive, sans nécessiter d'opération longue et délicate, sans changement d'instrumentation, ceci dans des conditions de fiabilité d'implantation et de contrôle aisé des. déplacements du filtre dans le corps.

C'est donc pour remplir ces exigences que l'invention propose un ensemble selon les revendications 1 et 2.

D'autres caractéristiques et avantages de l'invention apparaîtront de la description qui va suivre faite en référence aux dessins annexés dans lesquels :
- la figure 1 est une vue schématique d'ensemble de l'essentiel des moyens de l'invention, avec un arrachement local,
- la figure 2 montre schématiquement en coupe longitudinale un filtre dans une utilisation temporaire,
- la figure 3 est une vue en coupe le long de la ligne III-III de la figure 1,
- les figures 4, 5 et 6 montrent trois phases successives d'utilisation des moyens de l'invention dans le cadre d'une utilisation temporaire (figure 5) puis définitive du filtre (figure 6),
- les figures 7 et 8 montrent deux variantes du filtre de la figure 1,
- la figure 9 montre une variante de réalisation de la liaison entre la tige et le filtre de l'ensemble de la figure 2,
- les figures 10 et 11 montrent respectivement une troisième et une quatrième variantes du filtre de l'invention, et
- les figures 12 et 13 montrent respectivement, en vue agrandie et partielle, les filtres des figures 10 et 11 disposés dans la gaine d'implantation, dans un état contraint radialement.

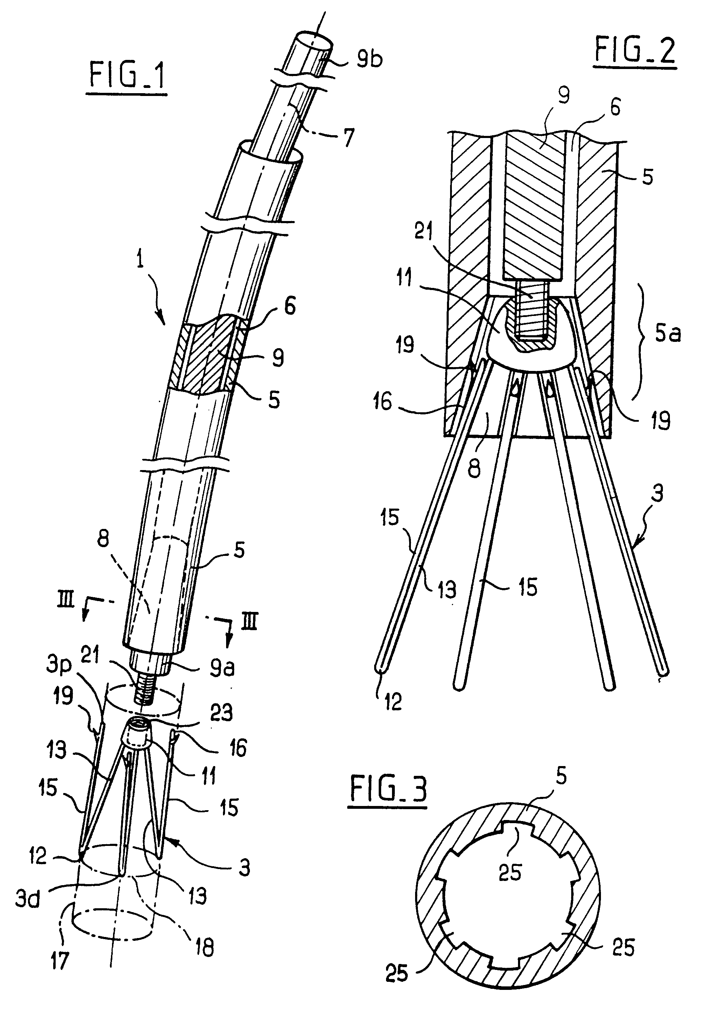

Sur la figure 1, on voit illustré un ensemble selon l'invention qui comprend un dispositif 1 d'implantation et d'utilisation d'un filtre sanguin 3, ce dispositif 1 étant connu dans ses caractéristiques générales (par exemple décrit dans FR-A-2 657 261 ou US-A-4 688 553).

En résumé, il comporte essentiellement une gaine extérieure 5 en matériau biocompatible, d'axe général longitudinal 7, à l'intérieur de laquelle peut être logé le filtre 3 dans un état radialement replié.

Pour agir sur le filtre (qui peut être réalisé en alliage métallique), une tige de manoeuvre 9, en matériau biocompatible, est montée axialement coulissante dans la gaine.

De manière en soi connue (voir par exemple les deux publications ci-dessus), le filtre 3, qui présente un axe 2, une extrémité proximale 3ₚ et une extrémité distale opposée 3_{d}, comporte une tête 11, par exemple en forme d'ogive, de laquelle sont issues des pattes 13 dont certaines au moins se prolongent à leur extrémité libre 12, par des appendices 15 de centrage et de maintien du filtre contre le vaisseau. Ces appendices reviennent en arrière, vers la tête 11, de telle sorte que les pattes ainsi constituées présentent une forme générale en épingle à cheveux.

Les pattes et les appendices auxquelles elles sont reliées pourront être constitués en fil métallique simple ou bouclé, ou comme de fines lames, de telle sorte que dans un état radialement non contraint le filtre 3 se développe, à l'endroit de ces pattes 13, en une corolle sensiblement conique, s'élargissant à partir de la tête 11 jusqu'à l'extrémité 3_{d}, tandis que les appendices 15 s'étendront en direction du côté de fermeture du cône, en étant sensiblement parallèle à la paroi du cylindre 17 engendré par une ligne génératrice parallèle à l'axe 7 du cône formé par le filtre et se déplaçant en décrivant la ligne 18 définissant le périmètre d'ouverture du filtre.

Ce filtre pouvant être utilisé en implantation définitive, il est équipé de moyens de fixation ou d'ancrage 19. Toutefois, selon une caractéristique importante de l'invention, les crochets 19 ont été ici prévus uniquement sur les appendices 15, du côté de leur extrémité libre 16, de manière à éviter tout risque d'ancrage non souhaité, dans une utilisation "temporaire", comme on le verra ci-après.

En relation justement avec une telle utilisation non définitive, l'extrémité distale 9a (destinée à être implantée le plus profondément dans le patient) de la tige 9 et la tête 11 du filtre sont agencées pour pouvoir être reliées entre elles, de manière amovible.

En l'espèce, ces moyens de liaison amovible consistent en des moyens mécaniques, par exemple en des moyens de vissage relatif.

Pour cela, l'extrémité 9a de la tige présente un prolongement axial fileté 21 adapté pour s'engager dans un taraudage 23 prévu axialement dans la tête 11 du filtre.

Sur la figure 2, qui montre d'ailleurs le filtre 3 et le dispositif 1 utilisés conjointement dans le cadre d'une implantation temporaire du filtre, on notera en outre la disposition des crochets 19 à l'intérieur de la gaine 5, en relation avec la disposition partiellement rentrée du filtre.

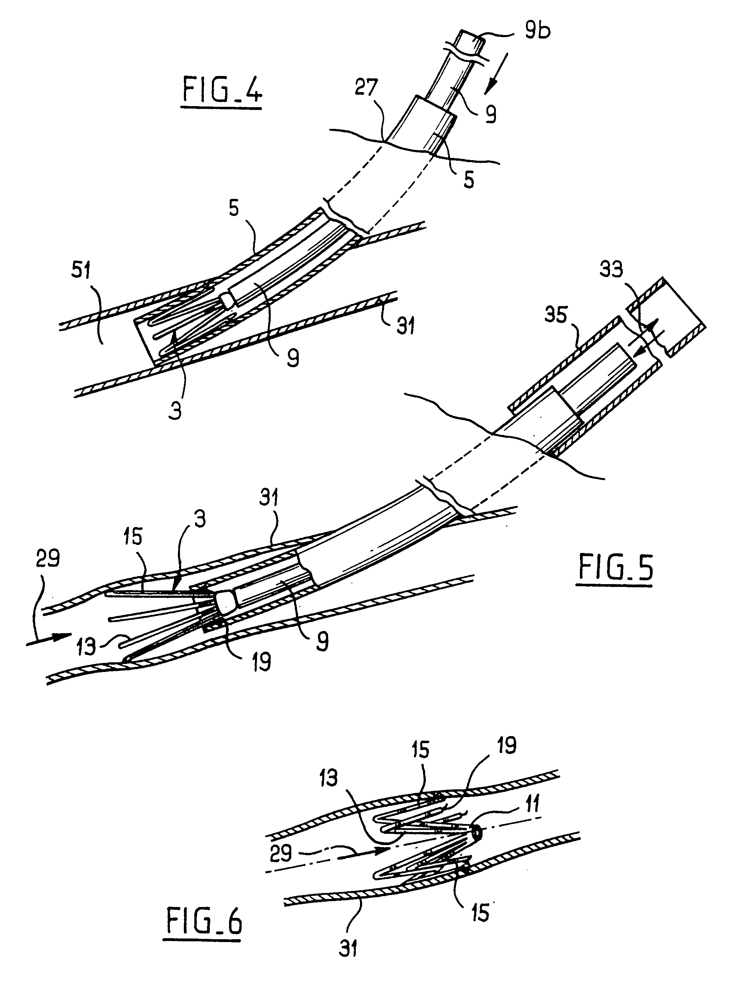

Si, comme dans l'exemple retenu, les moyens de liaison tige/filtre consistent en des moyens de vissage relatif, il a par ailleurs été prévu dans l'invention que la gaine présente, à sa partie extrême distale repérée par la zone 5a, une surface intérieure crantée. Comme représenté sur la figure 3, une série de crans intérieurs 25 en forme de fentes sensiblement parallèles à l'axe 7 de la gaine peut ainsi être prévue pour que chaque fente reçoive l'un des appendices 15 en agissant sur lui pour constituer des moyens de retenue en rotation du filtre lorsque le praticien va agir sur la tige 9 pour la dévisser en vue du passage d'une implantation temporaire à une implantation définitive. De préférence, on prévoira autant de crans 25 que d'appendices, l'élasticité naturelle radiale vers l'extérieur des appendices favorisant leur engagement dans les crans.

Au vu de la figure 2, on notera encore que la zone 5a distale de la gaine tubulaire pourra présenter un passage interne axial 8 de diamètre élargi par rapport à celui 6 situé plus en amont, de manière à favoriser un bon positionnement de la tête du filtre et un diamètre d'ouverture suffisant pour l'épanouissement de ce filtre en position d'implantation temporaire comme sur cette figure 2.

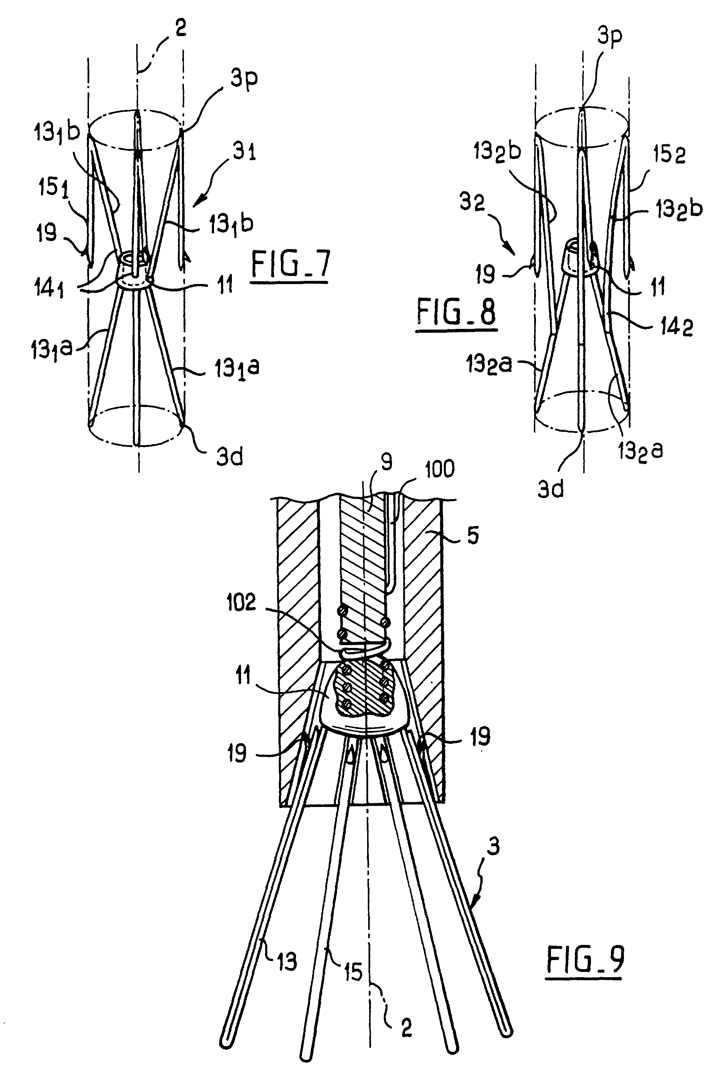

En se reportant maintenant aux figures 7 et 8, une première variante 3₁ et une seconde variante 3₂ du filtre de l'invention sont représentées. Elles comprennent, comme le filtre 3, une partie de filtration sensiblement conique, dépourvue de moyens d'ancrage au vaisseau et formée par la tête 11 (ici pourvue de moyens de liaison avec la tige 9) de laquelle sont issues des premières pattes 13₁a, 13₂a sensiblement rectilignes qui s'étendent jusque vers l'extrémité distale 3_{d} du filtre.

Contrairement au filtre 3, les filtres 3₁ et 3₂ comprennent chacun une série de secondes pattes 13₁b et 13₂b dont certaines au moins sont prolongées par les appendices 15₁ et 15₂ pour le maintien en place et le centrage respectivement des filtres 3₁ et 3₂ dans le vaisseau. Ces appendices comportent des moyens de fixation 19 du filtre contre la paroi du vaisseau, ces crochets 19 étant écartés de l'extrémité 3_{d} du filtre. Les secondes pattes et leurs appendices présentent une forme générale en épingle à cheveux et pourront être formés d'une seule pièce.

D'après la figure 7, les pattes 13₁b du filtre 3₁ sont fixées à une extrémité de fixation 14₁, à la partie filtrante (au niveau de la tête 11). Elles s'étendent depuis cette tête jusqu'à l'extrémité 3ₚ du filtre, de telle sorte que, dans leur état radialement non contraint, elles définissent une seconde corolle sensiblement conique, la première corolle de filtration et la seconde corolle étant tête-bêche .

D'après la figure 8, chaque patte 13₂b est fixée sur une patte 13₂a, en une portion ou zone de cette patte écartée de ses extrémités opposées (par exemple sensiblement en son milieu) de telle sorte que, dans leur état non contraint, les pattes 13₂b s'étendent depuis leur extrémité de fixation 14₂ aux pattes 13₂a suivant une seconde corolle ou une surface tubulaire allant en s'évasant, d'axe 2 (qui entoure la tête 11).

Les périmètres d'ouverture des première et seconde corolles pourront être sensiblement égaux (la seconde corolle filtrant également le sang qui la traverse).

Dans l'état non contraint des moyens de maintien, les appendices 15₁ et 15₂ s'étendent, ici sensiblement sur toute leur longueur, sensiblement parallèlement à l'axe 2 en définissant une surface tubulaire de section sensiblement égale à celle du vaisseau pour que ces appendices viennent au contact de la paroi du vaisseau et y fixent le filtre. Ces appendices sont dirigés en direction de l'extrémité 3_{d} du filtre, ainsi la seconde corolle s'étend à l'intérieur de la surface tubulaire définie par les appendices.

En utilisation temporaire des filtres 3₁ et 3₂, les appendices porteurs des crochets 19 et également ici les secondes pattes seront maintenus dans la gaine dans un état radialement contraint.

Les appendices et les secondes pattes pourront être constitués par des fils métalliques ronds ou plats, les secondes pattes étant par exemple soudées sur la première corolle.

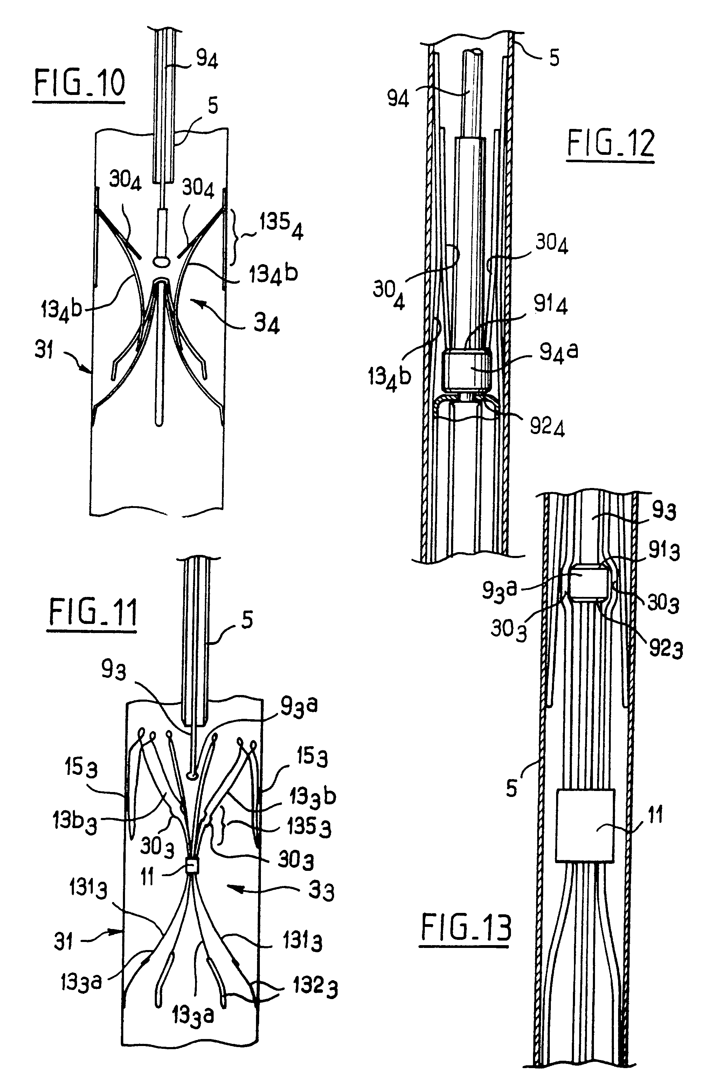

Le filtre 3₃ de la figure 11 se distingue de celui de la figure 7 en particulier en ce que chaque seconde patte 13₃b et son appendice 15₃ sont réalisés, de préférence en une seule pièce, par un fil souple replié sur lui-même en boucle(s) et dont les deux extrémités opposées, rapprochées l'une de l'autres, sont fixées au niveau de la tête 11 (pour plus de détails sur la constitution de ces pattes 13₃b et de leurs appendices, on pourra se reporter à US 5 344 427). Quant à certaines au moins des pattes 13₃a, elles comportent une partie principale 131₃ en fil raccordée à la tête et qui se prolonge par une partie distale 132₃ à contour non agressif vis-à-vis du vaisseau et de section supérieure à celle du fil pour augmenter la surface d'appui du filtre contre la paroi en ralentissant l'emprisonnement de ces parties distales par les tissus du vaisseau.

De plus, les filtres 3₃ de la figure 11 et 3₄ de la figure 10 se distinguent respectivement des filtres 3₁ et 3₂ en ce que les moyens de liaison amovible comprennent ici des moyens de serrage, ou de blocage axial de la tige, qui sont prévus sur les secondes pattes 13₃b (filtre 3₃) et 13₄b (filtre 3₄) en une zone 135₃, 135₄ intermédiaire entre la tête 11 et l'extrémité proximale du filtre (donc écartée des extrémités opposées du filtre). Ces moyens de serrage coopèrent avec des moyens complémentaires de la tige qui comprennent au moins une butée coopérante. Figure 11, les appendices 15₃ sont seulement sur une partie de leur longueur parallèles à l'axe 2.

A la figure 13, la tige 9₃ se termine par une extrémité 9₃a renflée qui présente deux butées 91₃ et 92₃, certaines au moins des pattes 13₃b présentant localement une portion recourbée ou créneau 30₃ pour le logement de cette extrémité renflée. Dans l'état contraint des moyens de maintien, lorsqu'ils sont contenus dans la gaine (utilisation temporaire du filtre), les créneaux 30₃ enserrent ou emprisonnent l'extrémité 9₃a de la tige, en venant en appui contre les butées pour empêcher tout déplacement axial de la tige par rapport au filtre. Dans l'utilisation définitive du filtre, les moyens de serrage, hors de la gaine, sont écartés des butées du fait de l'expansion radiale des secondes pattes.

Aux figures 10 et 12, certaines au moins des secondes pattes 13₄b portent des languettes flexibles 30₄ qui font saillie localement par rapport aux pattes pour bloquer l'extrémité 9₃a. Ces languettes s'étendent sur l'essentiel de leur longueur le long des pattes auxquelles elles sont liées et s'en écartent vers une de leur extrémité,libre,pour venir en appui, en utilisation temporaire du filtre (lorsque les pattes 13₄b sont contraintes dans la gaine) contre une butée 91₄ de l'extrémité renflée de la tige, tandis qu'une seconde butée 92₄ de cette extrémité renflée vient en appui contre la tête 11 du filtre. Ainsi, la tête du filtre coopère avec les languettes pour le blocage de la tige. Dans la position non contrainte des pattes 13₄b, les languettes sont écartées radialement de la tige qui est ainsi libérée.

On notera encore d'après les figures 10 à 13 que la tige présente ici un faible diamètre extérieur par rapport au diamètre intérieur de la gaine de telle sorte que les secondes pattes et leur appendices soient disposées entre la tige et la gaine dans l'utilisation temporaire du filtre.

Intéressons nous maintenant aux figures 4, 5 et 6 pour décrire un mode possible d'utilisation du dispositif 1 pour l'implantation du filtre 3.

Pour la technique générale d'implantation, on pourra se reporter en particulier aux publications précités ou encore au brevet US-A-4 990 156, étant précisé qu'après avoir ménagé une incision 27, on forme une voie d'accès jusqu'à la zone 51 d'implantation du filtre, puis, on introduit la gaine introductrice 5 pour qu'elle s'étende le long de la voie d'accès, son extrémité proximale ressortant du corps du patient et son extrémité distale étant dans la zone 51. En l'espèce, la mise en place du filtre, dans un état contraint, s'effectue ensuite à partir de l'extrémité proximale 5b de la gaine, le filtre 3 étant poussé le long de ce cathéter par la tige 9 liée au filtre jusqu'à ce qu'il soit positionné dans l'extrémité distale de la gaine (voir figure 4).

A la figure 5, par un déplacement relatif de la gaine par rapport à la tige, les pattes 13 et leurs appendices 15 du filtre sont en partie sortis de la gaine. Le filtre est bien entendu toujours lié à sa tige de manoeuvre 9 dont l'extrémité proximale 9b ressort toujours du corps du patient pour pouvoir être aisément manoeuvrée par le praticien.

La figure 5 montre le dispositif dans le cadre d'une filtration temporaire, le filtre étant alors comme dans sa position de la figure 2 où la partie de sa corolle située à l'extérieur de la gaine s'épanouit de sorte à occuper l'essentiel du diamètre du vaisseau en jouant son rôle de filtration, face au flux sanguin schématisé par la flèche 29. Bien entendu, dans cette position, les moyens d'ancrage 19 sont toujours logés à l'intérieur de la gaine, la partie des appendices 15 située encore à l'intérieur de cette gaine étant ici contrainte le long des pattes, suivant sensiblement la même configuration en corolle.

Si, au bout de quelques jours à quelques semaines, le praticien juge que les risques d'embolie ont suffisamment diminué, il peut alors retirer l'ensemble du dispositif, y compris le filtre. Pour cela, il lui suffit de tirer vers l'arrière la gaine (flèche 33), en retirant donc en même temps la tige et le filtre.

Si, par contre le praticien s'aperçoit qu'une implantation définitive du filtre 3 est préférable, il lui suffit alors de maintenir la gaine 5 toujours implantée et d'agir sur l'extrémité proximale 9b de la tige 9 pour dévisser celle-ci de la tête du filtre, lequel est empêché de tourner par les crans 25. Parallèlement, ou juste après, il agit à nouveau sur la gaine en la remontant par rapport à la tige 9 maintenue fixe, jusqu'à ce que les appendices 15 se déploient latéralement dans leur intégralité et que les crans 19 viennent s'engager dans la paroi du vaisseau, comme montré sur la figure 6 où l'on peut noter que les moyens 15 définissent une surface tubulaire sensiblement cylindrique centrée dans l'axe 33 du vaisseau 31 contre lequel ils s'appliquent, les moyens de filtration 11, 13 s'étendant à l'intérieur de cette surface pour retenir les éventuels caillots, tout en laissant circuler le sang dont le sens de circulation est toujours schématisé par la flèche 29.

Une fois le filtre ainsi positionné, le praticien peut bien entendu retirer la gaine et la tige 9 (séparée du filtre) par leur voie d'implantation, le filtre étant laissé à demeure.

Il est clair que d'autres variantes de réalisation de l'invention pourraient être imaginées. Ainsi, d'après la figure 9, la liaison séparable filtre/tige pourrait être assurée par au moins un fil 100, ou moyen équivalent, fixé à la fois à la tige 9, le long de laquelle il s'étend, et au filtre (à sa tête 11) pour les maintenir réunis dans une utilisation temporaire du filtre. Ce fil, par exemple en acier inoxydable, est recouvert d'une pellicule isolante (notamment en Teflon) sauf dans sa portion 102 assurant la liaison entre la tige et le filtre. Pour une utilisation définitive du filtre, ce fil peut être détruit localement ou coupé à l'endroit de la jonction 102, le praticien agissant à distance. Pour cela, on connecte l'extrémité proximale du fil à une source de courant disposée à l'extérieur du corps du patient. Le passage du courant dans la portion 102 qui est en contact direct avec le sang provoque sa désagrégation electrolytique entraînant la séparation du filtre et de la tige (pour plus de détails on pourra se reporter à US 5 122 136). En variante et tel que détaillé dans US 5 108 407, on pourrait utiliser une fibre optique (par exemple en quartz et recouverte d'une enveloppe en silice) qui s'étend le long de la tige en étant reliée, à son extrémité distale, à un connecteur métallique propre à être chauffé par une énergie lumineuse. Ce connecteur (qui peut consister en l'extrémité distale de la tige) est lié à la tête du filtre par une substance adhésive propre à se décomposer ou à perdre ses qualités d'adhésif à une température déterminée (fonction de cette substance et choisie supérieure à la température du corps du patient). Pour séparer le filtre de la tige, on émet un rayonnement (de préférence un rayon laser), via la fibre optique, jusqu'au connecteur qui chauffe alors et amène l'adhésif à la température à laquelle le filtre se sépare de la tige 9.

On pourrait également retenir une liaison mécanique à "baïonette" ou un moyen biologiquement résorbable qui se résorberait au contact d'un produit déterminé, biocompatible.

Quant au filtre, il pourrait s'agir du filtre de la demande FR 92 09 845 (filtre à pattes triangulées) ou encore de celui de la demande FR 92 15 774 avec des appendices conformés en zig-zag.

Pour la mise en place du filtre, on pourrait éventuellement utiliser un cathéter supplémentaire 35 dans lequel pourrait glisser la gaine 5, de manière à faciliter son introduction veineuse et les opérations de manoeuvre du dispositif 1 (figure 5).

## Revendications

1. Ensemble médical comprenant un filtre sanguin expansible dans un vaisseau (31) d'un patient, et un dispositif d'implantation vasculaire du filtre de manière temporaire ou définitive, dans lequel :
- le filtre présente des extrémités proximale (3p) et distale (3d) et comprend :
. des moyens de filtration qui comportent une tête (11) de laquelle sont issues des premières pattes (13₁a, 13₂a) expansibles radialement suivant sensiblement une première corolle, dans un état radialement non contraint,
. des moyens (15₁, 15₂, 15₃) de maintien en place du filtre par rapport au vaisseau, ces moyens de maintien comprenant des secondes pattes radialement expansibles, s'étendant, depuis la tête du filtre vers l'extrémité proximale de celui-ci, sensiblement suivant une seconde corolle dans leur état radialement expansé, les première et seconde corolles étant disposées tête-bêche, et
. des moyens d'ancrage (19) portés par les moyens de maintien, à l'écart de l'extrémité distale du filtre, de telle sorte qu'ils viennent au contact d'une paroi du vaisseau en utilisation définitive du filtre dans le vaisseau,
- et, ledit dispositif comprend :
. une gaine d'implantation (5) dans laquelle le filtre est disposé dans un état radialement replié, pour son implantation, ladite gaine présentant une extrémité proximale et une extrémité distale (5a),
. et, une tige (9) manoeuvrable depuis l'extrémité proximale de la gaine dans laquelle la tige est montée glissante, pour manoeuvrer le filtre en poussant ses moyens de filtration, au moins en partie hors de l'extrémité distale de la gaine, dans une utilisation temporaire du filtre,
l'ensemble se caractérisant en ce que :
- la tige de manoeuvre et le filtre comportent des moyens (30₃, 30₄, 91₃, 92₃, 91₄) de liaison amovible pour une liaison séparable entre eux, autorisant un déplacement axial du filtre dans deux sens opposés par rapport à la gaine, ceci dans l'utilisation temporaire du filtre alors situé à l'extrémité distale de la gaine, de manière que ses moyens d'ancrage (19) y soient contenus, tandis que ses moyens de filtration sont au moins en partie radialement expansés dans le vaisseau,
- lesdits moyens de liaison amovible comportent une première partie située vers l'extrémité distale de la tige et une seconde partie complémentaire située sur la tête (11) du filtre ou sur une zone des moyens de maintien du filtre intermédiaire entre ses extrémités proximale et distale, et
- si la seconde partie complémentaire de liaison est située sur la tête (11) du filtre, alors ladite liaison séparable est assurée par une fixation des moyens de liaison à la fois à la tige (9) et à la tête du filtre pour les maintenir réunis dans ladite utilisation temporaire du filtre.

2. Ensemble médical comprenant un filtre sanguin expansible dans un vaisseau (31) d'un patient, et un dispositif d'implantation vasculaire du filtre de manière temporaire ou définitive, dans lequel :
- le filtre présente des extrémités proximale (3p) et distale (3d) et comprend :
. des moyens de filtration qui comportent une tête (11) de laquelle sont issues des pattes (13; 13₁a, 13₂a, 13₁b, 13₂b) expansibles radialement dans un état radialement non contraint,
. des moyens (15) de maintien en place du filtre par rapport au vaisseau, ces moyens de maintien comprenant des appendices de centrage (15, 15₁, 15₂) prolongeant certaines au moins desdites pattes (13 ; 13₁b, 13₂b), les appendices définissant une surface sensiblement tubulaire dans l'état radialement expansé, non contraint, des pattes, pour être sensiblement parallèles à une paroi du vaisseau lorsque le filtre y est positionné, les pattes que prolongent des appendices étant situées à l'intérieur de cette surface tubulaire, et
. des moyens d'ancrage (19) portés par les appendices, à l'écart de l'extrémité distale du filtre, de telle sorte qu'ils viennent au contact de la paroi du vaisseau en utilisation définitive du filtre dans le vaisseau,
- et, ledit dispositif comprend :
. une gaine d'implantation (5) dans laquelle le filtre est disposé dans un état radialement replié, pour son implantation, ladite gaine présentant une extrémité proximale et une extrémité distale (5a),
. et une tige (9) manoeuvrable depuis l'extrémité proximale de la gaine dans laquelle la tige est montée glissante, pour manoeuvrer le filtre en poussant ses moyens de filtration, au moins en partie, hors de l'extrémité distale de la gaine, dans une utilisation temporaire du filtre,
l'ensemble se caractérisant en ce que :
- la tige de manoeuvre et le filtre comportent des moyens (21, 102) de liaison amovible pour une liaison séparable entre eux, autorisant un déplacement axial du filtre dans deux sens opposés par rapport à la gaine, ceci dans l'utilisation temporaire du filtre alors situé à l'extrémité distale de la gaine, de manière que ses moyens d'ancrage (19) y soient contenus, tandis que ses moyens de filtration sont au moins en partie radialement expansés dans le vaisseau,
- lesdits moyens de liaison amovible comportent une première partie située vers l'extrémité distale de la tige et une seconde partie complémentaire située sur la tête (11) du filtre, et
- cette seconde partie complémentaire de liaison étant ainsi située, ladite liaison séparable est assurée par une fixation des moyens de liaison à la fois à la tige (9) et à la tête du filtre pour les maintenir réunis dans ladite utilisation temporaire du filtre.

3. Ensemble selon la revendication 1 ou la revendication 2, **caractérisé en ce que** :
- les moyens de fixation amovible (100, 102) portés par la tige et le filtre sont des moyens électriques de fixation,
- les moyens électriques de fixation comprennent un fil électrique (100) qui présente une portion (102) fixée à la tige et au filtre pour les lier dans une utilisation temporaire du filtre, la portion étant adaptée pour se décomposer au contact du sang, lorsqu'elle est traversée par un courant électrique circulant à travers ledit fil, pour libérer le filtre de la tige.

4. Ensemble selon l'une quelconque des revendications 1 ou 2, **caractérisé en ce que** la gaine (5) comporte intérieurement, vers son extrémité distale (5a), des crans (25) anti-rotation propres à coopérer avec ledit filtre pour l'empêcher de tourner par rapport à la gaine et à la tige lorsqu'il est disposé dans la gaine, au moins en partie.

5. Ensemble selon la revendication 1, **caractérisé en ce que** :
- la tige (9₃, 9₄) présente un faible diamètre extérieur par rapport au diamètre intérieur de la gaine (5), de telle sorte que lesdites secondes pattes sont situées entre la tige et la gaine, dans l'utilisation temporaire du filtre et lorsque le filtre est entièrement dans la gaine.

6. Ensemble selon la revendication 1, **caractérisé en ce que** les moyens de fixation amovible comprennent des moyens de serrage (30₃, 30₄) pour le blocage axial de la tige par rapport au filtre, ces moyens de serrage étant portés par certaines au moins des secondes pattes, de telle sorte que :
- dans l'utilisation temporaire du filtre, les moyens de serrage soient contenus à l'intérieur de la gaine et enserrent au moins une butée (91₃, 92₃, 91₄) prévue sur ladite tige pour la bloquer axialement vis-à-vis du filtre, et
- dans l'utilisation définitive du filtre, l'expansion radiale des secondes pattes provoque l'écartement radial des moyens de serrage vis-à-vis de la butée, en autorisant ainsi la séparation entre le filtre et la tige.

7. Ensemble selon la revendication 6, **caractérisé en ce que** lesdits moyens de serrage comportent des languettes (30₄) liées à certaines au moins des secondes pattes, ces languettes bloquant la butée (9₄a) de la tige contre la tête du filtre, dans l'état contraint des secondes pattes, lorsque le filtre est dans la gaine.

8. Ensemble selon la revendication 1, **caractérisé en ce que** la seconde partie complémentaire de liaison est située sur une zone des moyens de maintien du filtre intermédiaire entre ses extrémités proximale et distale et certaines au moins desdites secondes pattes présentent localement un créneau (30₃) pour le logement de ladite butée (9₃a) de la tige, lesdits créneaux étant adaptés pour enserrer la butée, dans l'état contraint des secondes pattes, en bloquant ainsi axialement la tige par rapport au filtre.

## Patentansprüche

1. Medizinischer Aufbau mit einem in einem Gefäß (31) eines Patienten ausdehnbaren Blutfilter und eine Gefäßimplantierungsvorrichtung des Filters von temporärer oder permanenter Art, in welchem:
- der Filter proximale (3p) und distale (3d) Enden hat und aufweist:
◆ Filtriermittel, welche einen Kopf (11) aufweisen, von dem erste Stützen (13₁a, 13₂a) ausgehen, die radial in einem radial nicht beschränkten Zustand im wesentlichen einer ersten Krone folgen,
◆ Haltemittel (15₁, 15₂, 15₃), die den Filter bezüglich des Gefäßes am Platz halten, wobei diese Haltemittel zweite radial ausdehnbare Stützen aufweisen, die sich von dem Kopf des Filters zu dem proximalen Ende desselben im wesentlichen gemäß einer zweiten Krone in ihrem radial ausgedehnten Zustand erstrecken, wobei die ersten und zweiten Kronen umgekehrt angeordnet sind, und
◆ Verankerungsmittel (19), die durch Haltemittel von dem distalen Ende des Filters im Abstand derart gehalten sind, daß sie mit einer Wand des Gefäßes bei permanenter Benutzung des Filters in dem Gefäß in Kontakt kommen,
- und die Vorrichtung aufweist:
◆ eine Implantierungshülse (5), in welcher der Filter in einem radial zurückgeklappten Zustand für seine Implantierung angeordnet ist, wobei die Hülse ein proximales Ende und ein distales Ende (5a) aufweist,
◆ und eine von dem proximalen Ende der Hülse aus manövrierbare Stange (9), wobei die Stange in der Hülse gleitend montiert ist, um den Filter beim Stoßen seiner Filtriermittel mindestens zum Teil aus dem distalen Ende der Hülse bei einer temporären Benutzung des Filters zu betätigen, wobei der Aufbau **dadurch gekennzeichnet ist, daß**:
- die Betätigungsstange und der Filter Verbindungsmittel (30₃, 30₄, 91₃, 92₃, 91₄) aufweisen, die für eine trennbare Verbindung untereinander lösbar sind, wodurch eine axiale Verschiebung des Filters in zwei entgegengesetzte Richtungen bezüglich der Hülse erlaubt wird, wobei sich der Filter bei seiner temporären Benutzung dann an dem distalen Ende der Hülse derart befindet, daß seine Verankerungsmittel (19) dort enthalten sind, während seine Filtriermittel mindestens zum Teil radial in dem Gefäß ausgedehnt sind,
- die lösbaren Verbindungsmittel einen ersten Teil aufweisen, der zu dem distalen Ende der Stange hin liegt und einen komplementären zweiten Teil, der an dem Kopf (11) des Filters oder auf einem Bereich der Haltemittel des Filters zwischen seinen proximalen und distalen Enden liegt, und
- wenn der zweite komplementäre Verbindungsteil an dem Kopf (11) des Filters liegt, dann diese trennbare Verbindung durch eine Befestigung der Verbindungsmittel gleichzeitig an der Stange (9) und an dem Kopf des Filters sichergestellt wird, um sie bei der temporären Benutzung des Filters verbunden zu halten.

2. Medizinischer Aufbau mit einem in einem Gefäß (31) eines Patienten ausdehnbaren Blutfilter und eine Gefäßimplantierungsvorrichtung des Filters von temporärer oder permanenter Art, in welchem:
- der Filter proximale Enden (3p) und distale (3d) hat und aufweist:
◆ Filtriermittel, die einen Kopf (11) aufweisen, von dem Stützen (13; 13₁a, 13₂a, 13₁b, 13₂b) ausgehen, die radial in einem radial nicht beschränkten Zustand ausdehnbar sind,
◆ Haltemittel (15), die den Filter bezüglich des Gefäßes am Platz halten, wobei die Haltemittel Zentrierungsansatzstücke (15, 15₁, 15₂) aufweisen, die mindestens einige bzw. mehrere der Stützen (13; 13₁b, 13₂b) verlängern, wobei die Ansatzstücke eine im wesentlichen röhrenförmige Oberfläche in radial ausgedehntem, nicht beschränktem Zustand der Stützen bestimmen, um im wesentlichen zu einer Wand des Gefäßes parallel zu sein, wenn der Filter dort positioniert ist, wobei die Stützen, welche Ansatzstücke verlängern, im wesentlichen im Inneren dieser röhrenförmigen Oberfläche liegen, und
◆ Verankerungsmittel (19), welche durch die Ansatzstücke im Abstand von dem distalen Ende des Filters derart getragen sind, daß sie bei permanenter Benutzung des Filters in dem Gefäß in Kontakt mit der Wand des Gefäßes kommen,
- und die Vorrichtung aufweist:
◆ eine Implantierungshülse (5), in welcher der Filter in einem radial zurückgeklappten Zustand für seine Implantierung angeordnet ist, wobei die Hülse ein proximales Ende und ein distales Ende (5a) aufweist,
◆ und eine von dem proximalen Ende der Hülse aus manövrierbare Stange (9), wobei die Stange in der Hülse gleitend montiert ist, um den Filter beim Stoßen seiner Filtriermittel mindestens zum Teil aus dem distalen Ende der Hülse heraus bei einer temporären Benutzung des Filters zu betätigen,
wobei der Aufbau **dadurch gekennzeichnet ist, daß**:
- die Betätigungsstange und der Filter für eine trennbare Verbindung untereinander lösbare Verbindungsmittel (21, 102) aufweisen, wodurch eine axiale Verschiebung des Filters in zwei entgegengesetzte Richtungen bezüglich der Hülse erlaubt wird, wobei dieser bei temporärer Benutzung des Filters dann an dem distalen Ende der Hülse derart liegt, daß seine Verankerungsmittel (19) dort enthalten sind, während seine Filtriermittel mindestens zum Teil radial in dem Gefäß ausgedehnt sind,
- die lösbaren Verbindungsmittel einen ersten Teil aufweisen, der zu dem distalen Ende der Stange hin liegt, und einen zweiten komplementären Teil, der an dem Kopf (11) des Filters liegt, und
- der zweite komplementäre Verbindungsteil derart angeordnet ist, daß die trennbare Verbindung durch eine Befestigung der Verbindungsmittel gleichzeitig an der Stange (9) und an dem Kopf des Filters sichergestellt ist, um sie bei der temporären Benutzung des Filters verbunden zu halten.

3. Aufbau nach Anspruch 1 oder Anspruch 2, **dadurch gekennzeichnet, daß**:
- die durch die Stange getragenen lösbaren Befestigungsmittel (100, 102) und der Filter elektrische Befestigungsmittel sind,
- die elektrischen Befestigungsmittel einen elektrischen Draht (100) aufweisen, der einen an der Stange und an dem Filter befestigten Teil (102) aufweist, um sie bei temporärer Benutzung des Filters zu verbinden, wobei der Teil geeignet ist, sich bei Kontakt des Blutes zu zersetzen, wenn er von einem elektrischen Strom durchquert wird, der durch den Draht zirkuliert, um den Filter von der Stange freizugeben.

4. Aufbau nach einem der Ansprüche 1 oder 2, **dadurch gekennzeichnet, daß** die Hülse (5) innen zu ihrem distalen Ende (5a) hin Antirotationseinkerbungen (25) aufweist, die geeignet sind, mit dem Filter zusammenzuwirken, um ihn daran zu hindern, bezüglich der Hülse und der Stange zu drehen, wenn er mindestens zum Teil in der Hülse angeordnet ist.

5. Aufbau nach Anspruch 1, **dadurch gekennzeichnet, daß**:
- die Stange (9₃, 9₄) einen geringen Außendurchmesser bezüglich des Innendurchmessers der Hülse (5) aufweist, derart, daß die zweiten Stützen bei temporärer Benutzung des Filters und wenn der Filter sich ganz in der Hülse befindet, zwischen der Stange und der Hülse liegen.

6. Aufbau nach Anspruch 1, **dadurch gekennzeichnet, daß** die lösbaren Befestigungsmittel Klemmittel (30₃, 30₄) zum axialen Blockieren der Stange bezüglich des Filters aufweisen, wobei diese Klemmittel durch einige mindestens der zweiten Stützen derart getragen sind, daß:
- bei temporärer Benutzung des Filters die Klemmittel im Inneren der Hülse enthalten sind und mindestens einen Anschlag (91₃, 92₃, 91₄) klemmen, der auf der Stange vorgesehen ist, um sie axial gegenüber dem Filter zu blockieren, und
- bei permanenter Benutzung des Filters die radiale Ausdehnung der zweiten Stützen den radialen Abstand der Klemmittel gegenüber dem Anschlag hervorruft, wodurch so die Trennung zwischen dem Filter und der Stange erlaubt wird.

7. Aufbau nach Anspruch 6, **dadurch gekennzeichnet, daß** die Klemmittel Zungen (30₄) aufweisen, die mit mindestens einigen der zweiten Stützen verbunden sind, wobei diese Zungen den Anschlag (9₄a) der Stange gegen den Kopf des Filters in dem beschränkten Zustand der zweiten Stützen blockieren, wenn sich der Filter in der Hülse befindet.

8. Aufbau nach Anspruch 1, **dadurch gekennzeichnet, daß** der zweite komplementäre Verbindungsteil auf einem Bereich der Haltemittel des Filters zwischen seinen proximalen und distalen Enden liegt und mindestens einige der zweiten Stützen örtlich eine zinnenförmige Zacke (30₃) für den Sitz des Anschlages (9₃a) der Stange aufweisen, wobei die Zacken geeignet sind, den Anschlag in dem beschränkten Zustand der zweiten Stützen einzuklemmen, wobei so die Stange bezüglich des Filters axial blockiert wird.

## Claims

1. A medical assembly comprising a blood filter expandable in a blood vessel (31) of a patient, and a vascular implantation device for implanting the blood filter, either temporarily or permanently, wherein :
- the blood filter has a proximal end (3p) and a distal end (3d), and comprises :
. filtering means comprising a head (11) from which extend first legs (13₁a, 13₂a) which are radially expandable for defining a first corolla, in a radially non-constrained state,
. maintaining means (15₁, 15₂, 15₃) for holding the blood filter in the blood vessel, the maintaining means comprising second radially expandable legs extending, from the filter head, towards the proximal end thereof, substantially along a second corolla, in a radially expanded state thereof, the first and second corollas been disposed top to bottom, and
. anchoring means (19) provided on the maintaining means, apart from the distal end of the blood filter, so that the anchoring means are in contact with a wall of the blood vessel, while the blood filter is permanently used in said blood vessel,
- and the vascular implantation device comprises :
. an implantation sheath (5) in which the blood filter is disposed in a radially constrained state, for its implantation, the implantation sheath having a proximal end and a distal end (5a),
. and a stem (9) movable from the proximal end of the implantation sheath in which the stem is adapted to slide, for manoeuvring the blood filter by pushing the filtering means at least partially out of the distal end of the implantation sheath, in a temporary use of the blood filter, the medical assembly being **characterized in that**:
- the manoeuvring stem and the blood filter have removable connecting means (30₃, 30₄, 91₃, 92₃, 91₄) for a removable connection therebetween, so that an axial movement of the blood filter along two opposite directions with respect to the implantation sheath is allowed, in a temporary use of the blood filter while said blood filter is located at the distal end of the implantation sheath, so that the anchoring means (19) are contained within the implantation sheath, while the filtration means are, at least partially, radially expanded in the blood vessel,
- the removable connecting means have a first part located towards the distal end of the manoeuvring stem and a second, complementary part located either on the head (11) of the blood filter or in a zone of the filter maintaining means intermediate between the proximal end and the distal end thereof, and
- if the second, complementary part is located at the head (11) of the filter, then the removable connection is obtained by fixing the connecting means to the manoeuvring stem (9) and to the filter head for maintaining them connected together, while the blood filter is temporarily used.

2. A medical assembly comprising a blood filter expandable in a blood vessel (31) of a patient, and a vascular implantation device for implanting the blood filter, either temporarily or permanently, wherein :
- the blood filter has a proximal end (3p) and a distal end (3d) and comprises :
. filtering means comprising a head (11) from which extend legs (13, 13₁a, 13₂a, 13₁b, 13₂b) which are radially expandable in a radially non-constrained state,
. maintaining means (15₁, 15₂, 15₃) for holding the blood filter in the blood vessel, the maintaining means comprising centring appendices (15, 15₁, 15₂) extending at least from of the legs (13, 13₁b, 13₂b), the appendices defining a substantially tubular surface in the radially expanded, non-constrained state of the legs for being orientated substantially parallel to a wall of the blood vessel where the blood filter is disposed, the legs which are extended by the appendices being located within said tubular surface, and,
. anchoring means (19) provided on the appendices apart from the distal end of the blood filter, so that the anchoring means are in contact with the wall of the blood vessel, while the blood filter is permanently used in said blood vessel,
- and the vascular implantation device comprises :
. an implantation sheath (5) in which the blood filter is disposed in a radially constrained state, for its implantation, the implantation sheath having a proximal end and a distal end (5a),
. and a stem (9) movable from the proximal end of the implantation sheath in which the stem is adapted to slide, for manoeuvring the blood filter by pushing the filtering means at least partially out of the distal end of the implantation sheath, in a temporary use of the blood filter, the medical assembly being **characterized in that**:
- the manoeuvring stem and the blood filter have removable connecting means (21, 102) for a removable connection therebetween, so that an axial movement of the blood filter along two opposite directions with respect to the implantation sheath is allowed, in a temporary use of the blood filter while said blood filter is located at the distal end of the implantation sheath, so that the anchoring means (19) are contained within the implantation sheath, while the filtration means are, at least partially, radially expanded in the blood vessel,
- the removable connecting means have a first part located towards the distal end of the manoeuvring stem and a second, complementary part located on the head (11) of the blood filter, and
- said second, complementary part being thus located, then the removable connection is obtained by fixing the connecting means to the manoeuvring stem (9) and to the filter head for maintaining them connected together, while the blood filter is temporarily used.

3. The assembly according to claim 1 or claim 2, **characterized in that**:
- the removable connecting means (100, 102) provided on the manoeuvring stem and on the blood filter are electrical connection means,
- the electrical connection means comprise an electrical wire (100) having a portion (102) fixed to the manoeuvring stem and the filter, for connecting them together in a temporary use of the blood filter, the portion being adapted for being decomposed where in contact with blood, further to the circulation of an electrical current in the electrical wire, for allowing the blood filter to be separated from the manoeuvring stem.

4. The assembly according to anyone of claims 1 or 2, **characterized in that** the implantation sheath (5) encloses, towards its distal end (5a), anti-rotation notches (25) adapted for co-operating with the blood filter for preventing it to rotate with respect to the implantation sheath and the manoeuvring stem, where the blood filter is at least partially disposed within the implantation sheath.

5. The assembly according to claim 1, **characterized in that**:
- the manoeuvring stem (9₃, 9₄) has an outer diameter which is less than the inner diameter of the implantation sheath (5) so that the second legs are located between the manoeuvring stem and the implantation sheath, as long as the blood filter is temporarily used and is entirely located within the implantation sheath.

6. The assembly according to claim 1, **characterized in that** the removable connecting means comprise squeezing means (30₃, 30₄) for axially blocking the manoeuvring stem with respect to the blood filter, the squeezing means being provided on at least some of the second legs, so that:
- in the temporary use of the blood filter, the squeezing means are contained within the manoeuvring sheath and squeeze at least a stop (91₃, 92₃, 91₄) provided on the manoeuvring stem for blocking it axially with respect to the blood filter, and
- in the permanent use of the blood filter, the radial expansion of the second legs induces a radial spacing apart of the squeezing means with respect to the stop, so that the separation between the blood filter and the manoeuvring stem is allowed.

7. The assembly according to claim 6, **characterized in that** the squeezing means comprise tongues (30₄) connected to at least some of the second legs, the tongues being adapted for blocking the stop (9₄a) of the manoeuvring stem against the head of the blood filter, in the radially constrained state of the second legs, while the blood filter is located within the implantation sheath.

8. The assembly according to claim 1, **characterized in that** the second, complementary part of the removable connecting means is located in a zone of the maintaining means of the blood filter intermediate between the proximal end and the distal end, and at least some of the second legs locally show an indentation (31₃) for receiving the stop (9₃a) of the manoeuvring stem, the indentations being adapted for squeezing the stop, in the constrained state of the second legs, and axially blocking thereby the manoeuvring stem with respect to the blood filter.
